(19)

**Europäisches Patentamt**

**European Patent Office**

**Office européen des brevets**

(11) **EP 4 198 997 A1**

(12) **EUROPEAN PATENT APPLICATION**

(43) Date of publication:
**21.06.2023 Bulletin 2023/25**

(21) Application number: **21215215.1**

(22) Date of filing: **16.12.2021**

(51) International Patent Classification (IPC):
***G16H 30/40*** *(2018.01)*

(52) Cooperative Patent Classification (CPC):
**G16H 30/40; G16H 50/20; G16H 50/50; G16H 50/70**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **GESSERT, Nils Thorben**
  **Eindhoven (NL)**
• **LOSSAU, Tanja**
  **Eindhoven (NL)**
• **PETERS, Jochen**
  **Eindhoven (NL)**

• **WEBER, Frank Michael**
  **Eindhoven (NL)**
• **WAECHTER-STEHLE, Irina**
  **Eindhoven (NL)**
• **EWALD, Arne**
  **Eindhoven (NL)**
• **GOOßEN, André**
  **Eindhoven (NL)**
• **GROTH, Alexandra**
  **Eindhoven (NL)**
• **WILD, Sebastian**
  **Eindhoven (NL)**

(74) Representative: **Philips Intellectual Property & Standards**
**High Tech Campus 52**
**5656 AG Eindhoven (NL)**

(54) **A COMPUTER IMPLEMENTED METHOD, A METHOD AND A SYSTEM**

(57) A computer implemented method of identifying changes in a subject's heart or an adjacent region over-time. The method comprising: receiving a set of imaging data relating to a subject's heart that has been obtained at a plurality of points in time; generating an anatomical model of the subject's heart for each of the images in the set of imaging data so as to provide a set of anatomical models of the subject's heart corresponding to the plurality of points in time; and aligning each of the anatomical models in the set of anatomical models relative to one another so as to provide a set of aligned data of the subject's heart. The aligned data are for identifying changes in at least one region of the subject's heart by comparing the anatomical models in the set of aligned anatomical models using a machine learning model.

FIG. 1

EP 4 198 997 A1

**Description**

FIELD OF THE INVENTION

A computer implemented method that identifies changes in a subject's heart over time, a method of identifying changes in a subject's heart over time and a system for identifying changes in a subject's heart over time.

BACKGROUND OF THE INVENTION

Heart failure (HF), also known as congestive heart failure (CHF) and (congestive) cardiac failure (CCF), is a relatively common set of manifestations caused by the failure of the heart's function as a pump supporting the blood flow through the body. Heart failure is a common, costly, chronic and potentially fatal condition. Being one of the leading causes of hospitalization as well as readmission amongst older adult, heart failure continues to be a major and growing public health problem. For number of patients, heart failure is a long-term condition that cannot be cured.

In heart failure, the heart is losing pump power. The heart is the driving force in the circulatory system, but it shares the responsibility for sufficient organ perfusion with the kidneys. Changes in cardiac performance are compensated by the kidneys. One of these compensatory mechanisms is to retain fluid to upregulate blood volume. This changes the hemodynamic status of the patient

In addition to diagnosing heart failure which is important to determine treatment(s) that can keep heart failure symptoms under control, predicting heart failure would provide number of benefits for (future) patients. For instance, such patients could be pre-emptively informed, coached such as to be able to take preventive action(s) or pre-emptively treated, and thereby avoiding or limiting hospitalization, readmission and/or possibly death.

Forecasting heart failure and early onset heart-failure prediction are challenging clinical problems. If patient's heart function is already compromised, measurements from ultrasound or other imaging modalities can provide insights through measures such as left ventricular ejection fraction. However, early signs of heart failure can be subtle and not detectable by single measurements making it difficult to forecast the condition.

Typically, biomarkers and derived measures are used as predictors for future heart failure. These can include BMI, age, gender, health behavior, heart-rate variability, and other clinical measures. Algorithms may be trained to predict and forecast heart failure based on these variables.

An issue with this approach is that the input data for the algorithm does not have enough heart-based anatomical information to make an accurate prediction.

SUMMARY OF THE INVENTION

Accordingly, there is a continued need for methods and systems for accurately, reliably and consistently predicting onset of heart failure of a subject.

In a first aspect of the invention, there is provided a computer implemented method of identifying changes in a subject's heart or an adjacent region(s) overtime, the method comprising: receiving a set of imaging data relating to a subject's heart, the set of imaging data comprised of images of the subject's heart obtained at a plurality of points in time; generating an anatomical model of the subject's heart for a plurality of the images in the set of imaging data so as to provide a set of anatomical models of the subject's heart corresponding to a plurality of points in time; and aligning a plurality of the anatomical models in the set of anatomical models relative to one another so as to provide a set of aligned data, wherein the set of aligned data can be used to identify changes in at least one region of the subject's heart by comparing data in the set of aligned data using a machine learning model.

Embodiments provide a method which is more sensitive to changes in a patient's heart condition and can more accurately determine changes that have occurred in the heart or an adjacent region (e.g. regions where changes can be identified by looking at patient's heart and the immediately surrounding portions) by comparison of longitudinal studies of a patient. Receiving input data that has been collected at a plurality of points in time has the advantage of allowing changes to parts of the heart and their progression to be identified rather than assessing a subject's heart function at a single point in time; however, often imaging of a patient taken over time cannot easily be compared. For example, it can be difficult to compare images due to large variations between investigations caused by the use of different devices, different acquisition parameters, and operator dependencies. The creation of a set of aligned data allows for more sensitive and accurate comparisons to be made between image data that has been collected over time. For example, when images are taken at a plurality of points in time, there will likely be different field of views, meaning that the images do not capture the identical areas. Without aligned data, it would be difficult to compare these images without a human manually inspecting each image and comparing it to previous images in order to identify developments. Alignment of the data (e.g. alignment of the anatomical models or mesh) enables the comparison of intensities in the images and interrogation of particular anatomical regions of interest. This in turn allows for smaller anatomical variations to be tracked, enabling earlier and more accurate heart-failure forecasting. The aligned data that is produced from the models may be produced from the anatomical models or from a derivative of the anatomical model. In an embodiment, alignment of the models may include sequentially aligning the models with the plurality of points in time.

The anatomical model is an anatomical representation of a plurality of (e.g. each, in some embodiments) of the images obtained across a plurality of time points. In an embodiment, each anatomical model may be based on a single image taken from a single time point. This may include generating a model for each of a plurality of images in a single study obtained at a single time point (and carrying this out for plural studies). In other embodiments, several models may be based on a plurality of images taken from a study obtained at a single time point. The anatomical model may be a "mesh" or "anatomical mesh", for example a mesh generated by a model-based segmentation. In certain embodiments, each model may be a model-based segmentation constructed from a 3D image (such as a 3D ultrasound image). A plurality of the images in the imaging data may be converted to models or meshes. In some embodiments, this may be each image in the imaging data.

Medical imaging data can include scans or frames of a particular anatomical region, including for example 2D, 3D, 2D+t or 3D+t medical imaging data (e.g. ultrasound imaging, computerized tomography (CT) imaging, magnetic resonance imaging (MRI)). The method can be applied to a collection of individual images or frames that form a sequence or a combination thereof.

In an embodiment, the computer implemented method can identify changes in at least one region of the subject's heart by comparing data in the set of aligned data using a machine learning model; and producing an output containing information relating to the subject's heart.

An output containing information relating to a subject or patient's heart can include an indication of changes or no change in a subject's heart, or may in some embodiments be a prediction relating to the onset of a cardiovascular condition. For example, the cardiovascular condition that the output relates to may be coronary artery disease, dilated cardiomyopathy or pulmonary stenosis. Producing an output containing information regarding the subject's heart is of benefit since it can be used by a medical practitioner to obtain information that would not have otherwise been easily obtainable. Having a machine learning model that takes into account small anatomical variations and which uses imaging taken over time and aligned using the abovementioned method allows for earlier and more accurate analysis, which then can be used in heart-failure forecasting.

The use of machine learning, through a machine learning engine or model, provides additional benefits as more subtle anatomical changes overtime can be detected. The machine learning model receives the inputs in the form of the aligned data (e.g. aligned models) and can use a machine learning engine to determine e.g. changes between the images overtime. The machine learning model may be trained such that the machine learning model is able to provide the output. In some embodiments, a training database may be provided which may contain training information including images and the associated outputs.

The processing using machine learning may comprise generating a feature vector or plural feature vectors based on a plurality of the (e.g. each, in some embodiments) anatomical model. In an embodiment, the method further comprises combining the vectors prior to analyzing the vectors. Combining the output vectors can be carried out using a number of different ways, such as stacking the vectors as a one-dimensional vector (*e.g.*

$$x \in \mathbb{R}^{NM}$$

(1D) where there are N vectors of length M) or multidimensional vectors (e.g. a 2D sequence of vec-

tors (e.g. $\in \mathbb{R}^{NxM} (2D))$ . Accordingly, in an embodiment of the computer implemented method, combining the vectors comprises combining the vectors to form a one-dimensional or multidimensional vector (e.g. a 2D sequence of vectors). The computer implemented method's ability, in some embodiments, to process multiple types of data including both image, signal and non-image data through time allows for significant improvements in accuracy to be made over previous attempts to predict disease onset or progression.

In a further embodiment, the computer implemented method can produce an output wherein the output is a prediction relating to the onset of a cardiovascular condition. Cardiovascular conditions can be fatal therefore outputting a prediction relating specifically to their onset is advantageous for managing a subject's health. The prediction may be a prediction as to the progression of the condition. Conditions can include heart failure, such as mitral-valve failure.

In an embodiment, the computer implemented method can be applied such that an anatomical model of a patient's entire heart is produced. Modelling of the entire heart enables changes to be identified in a greater number of regions of the heart than if modelling only takes place for a part of the heart and allows for additional assessment of adjacent anatomical regions.

As such, in one embodiment, the method further comprises identifying changes in at least one region of the subject's heart by comparing data in the set of aligned data using the machine learning model; and generating an output containing information relating to the subject's heart. Comparing data in the set of aligned data may comprise comparing some data or all of the data in the aligned data.

In an embodiment, the steps of aligning a plurality (e.g. each, in some embodiments) of the anatomical models in the set of anatomical models and identifying changes in at least one region of the subject's heart by comparing data in the set of aligned data using a machine learning model comprises: extracting data relating to the at least one region of the subject's heart from each of a plurality of the anatomical models (e.g. each anatomical model); generating a graph representative of the extracted data for each of the plurality of anatomical models (e.g. each anatomical model); and comparing the graphs for each of the plurality of anatomical models (e.g. each anatomical model)..

In this way, some or all of the anatomical model or meshes are converted into a graph that can encode key features and information derived from the anatomical model, and therefore the mesh. The comparison of some or all of graphs that are produced from anatomical models allows changes to be identified in various regions of the heart.

Comparing the graphs may comprise generating a feature vector or plural feature vectors based on each graph, with each vector encoding data relating to at least one region from each anatomical model. In some embodiments, a plurality of or each vector can then be processed by a recurrent module. This can produce features representing or defining the heart's state at a particular point in time (based on a single frame or based on per-study (i.e. a collection of images or frames taken during a single study at a single time point) features). In some embodiments, these features can then be further processed by an additional recurrent module to determine long-term (i.e. longitudinal) changes or development and to provide the output.

The comparison of the graphs is carried out by the machine learning model. This model may use or comprise a convolutional neural network (e.g. a graph- or mesh-based CNN). The model, such as a graph based CNN can extract anatomical features from each image or frame. The machine learning model may further use or comprise recurrent processing units. In this context, a recurrent processing unit is able to process sequential data. The recurrent processing units are able to process data that has been taken at a plurality of points in time. The recurrent processing units can be gated recurrent units (GRUs), long short-term memory (LSTM) cells, or other recurrent models.

The aligned data that is produced from the anatomical models may be produced from the anatomical models or from a derivative of the anatomical models.

In one embodiment, the step of aligning a plurality of (e.g. each) anatomical models in the set of anatomical models relative to one another is carried out prior to extracting the data relating to at least one region of the heart such that the extracted data is extracted aligned data. Thus, the models are aligned so as to produce a set of aligned models and data can then be extracted from the aligned models. Accordingly, in some embodiments, the computer implemented method comprises identifying changes in a subject's heart or an adjacent region over time, the method comprising: receiving a set of imaging data relating to a subject's heart, the set of imaging data comprised of images of the subject's heart obtained at a plurality of points in time; generating an anatomical model of the subject's heart for a plurality (e.g. each) of the images in the set of imaging data so as to provide a set of anatomical models of the subject's heart corresponding to the plurality of points in time; and

aligning a plurality of (e.g. each) of the anatomical models in the set of anatomical models relative to one another so as to provide a set of aligned models of the subject's heart, wherein the set of aligned models are for identifying changes in at least one region of the subject's heart by comparing the models in the set of aligned anatomical models using a machine learning model.

Alignment of the anatomical models (e.g. a mesh) provides a consistent field-of-view (FOV) and enables the comparison of intensities in the images and interrogation of particular anatomical regions of interest. This in turn allows for smaller anatomical variations to be tracked, enabling earlier and more accurate heart-failure forecasting.

Alignment of the anatomical model which is produced from the images can comprise alignment of the models in space so that these parts can be compared. This can be alignment based on anatomical coordinate frame, in some embodiments. Alignment can be of the whole of the data (e.g. the model or a representation of the model) or may be a part of the data, such as a region of interest.

In an embodiment, aligning a plurality of (e.g. each) of the anatomical models in the set of anatomical models relative to one another so as to provide the set of aligned data of the subject's heart comprises: defining a coordinate frame for a plurality of (e.g. each) anatomical model based on at least one identifiable anatomical feature common in each anatomical model; and aligning the representations by aligning the coordinate frame of the plurality of (e.g. each) of the anatomical models. Thus, the aligned data in this embodiment is set of aligned anatomical models.

Simultaneous processing of longitudinal ultrasound studies requires a consistent field-of-view (FOV), intensity normalization, and overlaying images onto a common coordinate system which is usually error-prone or even infeasible. The use of coordinate frames allows for the alignment of specific regions of interest of the heart (or adjacent region(s)), thereby allowing for the more accurate and more sensitive assessments to occur.

The anatomical coordinate frame is a coordinate frame that enables reference to be made to coordinates in 3D space that correspond to at least one, and preferably multiple parts of the body. In this method, the coordinate frame may be aligned to a particular feature or particular features of the heart, such as landmark features. Anatomical landmarks that are common to the heart can be identified and used for alignment. The use of the anatomical landmarks provides a useful tool for comparison of the different images that would otherwise have an inconsistent field of view.

In an alternative embodiment, the step of aligning a plurality of (e.g. each) anatomical models in the set of anatomical models relative to one another is carried out after the step of extracting the data relating to at least one region of the heart, such that the extracted data is subsequently aligned. This may take part during the step of producing the graphs, in some embodiments. For example, this may comprise producing a graph directly from a plurality of (e.g. each) anatomical models such that alignment of the anatomical models occurs alongside the identification and production of one of: geometric features, anatomical region codes and neighboring image intensities for the graph.

In an embodiment of the computer implemented method, the data relating to the at least one region from a plurality of (e.g. each) anatomical models comprises at least one of: coordinates in a coordinate frame associated with the region of the subject's heart, geometric features, anatomical region codes and/or image intensities.

By mapping image intensities onto the graph, image information can be made use of while avoiding inconsistencies in the raw image sequences. The usage of these processed image intensities is particularly relevant for scenarios where the early onset of heart failure is not directly entering the coordinates of the adapted heart model, but only manifests itself as subtle changes of the intensities around the heart model position (e.g. thickening of wall structures).

The mapping of image intensities facilitates the machine learning component present and allows long-term studies to be processed.

The geometric features include the relative positions of different areas of the heart and may include factors such as size, spacing and relative angles.

The anatomical region codes are codes that are assigned to different parts of the heart. This enables regions of the heart to be clearly identified during processing.

In an embodiment of the computer implemented method, the machine learning model identifies changes in at least one region of the subject's heart by comparing the graphs for each of the plurality of the (e.g. each) anatomical models using a recurrent processing unit. Examples of recurrent processing units that may be used are gated recurrent units and long short-term memory cells.

In an embodiment of the computer implemented method, the recurrent processing unit performs temporal processing within a cardiac cycle; and/or identifies changes of motion trajectories. The temporal processing within a cycle allows for irregularities to be identified and the analysis of motion trajectories further acts to increase the range of analysis that is being carried out when changes are being identified.

Alternatively, in some embodiments, the machine learning model identifies changes in at least one region of the subject's heart by comparing the graphs for each anatomical model by using an attention-based machine learning model, such as transformers. Examples of such transformers is set out in Vaswani, A., Shazeer, N., Parmar, N., Uszkoreit, J., Jones, L., Gomez, A. N., ... & Polosukhin, I. (2017). Attention is all you need. In Advances in neural information processing systems (pp.

5998-6008), which is incorporated herein in its entirety by reference.

In an embodiment, the output comprises an indication of the input data or a part of the input data on which the output has been based. This may be a diagram representing at least a subsection of the heart. The output (e.g. a diagram of a subsection of the heart) may contain information regarding the input data that was most important for producing the prediction regarding a patient's heart. In other words, it may identify the particular region of interest in the heart or may highlight the image(s) or time point(s) (e.g. studies) that provided the largest contribution to the output or prediction. In some embodiments, particular regions within particular images (or frames) may be identified with an indicator of relevance. In some embodiments, the output is a saliency map. A saliency map may also be used to generate the outputs, in some embodiments.

In an embodiment, the images comprise ultrasound images. In a further embodiment, the ultrasound images are three dimensional ultrasound images. The use of ultrasound, and in particular 3D ultrasound images allows for a non-invasive imaging technique to be used when obtaining data. This technique is widely used therefore the collection of heart data will not pose a significant challenge to medical practitioners. There is therefore also likely to be a pool of existing ultrasound images in a patient's medical history for those patients suffering or at higher risk of suffering from cardiovascular conditions such as heart failure. Thus, this method can provide outputs using existing data.

In an embodiment, the method further comprises training the machine learning model (the machine learning model is therefore a trained machine learning model). This may be carried out prior to the comparison step. Training can occur prior to or as part of the identification using the machine learning model. The model may be trained using a dataset comprising heart failure patient data with multiple images (or studies) at a plurality of points in time being available before heart failure was first diagnosed in the patient. The computer implemented method is able to process several types of medical imaging data. There is therefore a large pool of potential training data which has been taken over time which may be used to train this machine learning model. In one embodiment, training is carried out using medical data duplets, each medical data duplet comprising: (i) a plurality of medical imaging images or frames for a respective patient; and (ii) disease diagnosis and/or progression data for the patient.

One advantage of the method is that no special data annotation is required for building the invention and training the machine learning model. Retrospective clinical data of heart-failure patients can be used by training the machine learning model on longitudinal studies that have been performed before heart failure was diagnosed in the patient.

In a second aspect, a method includes identifying changes in a subject's heart or an adjacent region over time, the method comprising: obtaining images of a subject's heart at a plurality of points in time to produce a set of imaging data; generating an anatomical model of the subject's heart for a plurality of (e.g. each) of the images in the set of imaging data so as to provide a set of anatomical models of the subject's heart corresponding to a plurality of points in time; and aligning a plurality of (e.g. each) of the anatomical models in the set of anatomical models relative to one another so as to provide a set of aligned data, wherein the set of aligned data is for identifying changes in at least one region of the subject's heart by comparing data in the set of aligned data using a machine learning model.

In an embodiment, the method includes identifying changes in at least one region of the subject's heart or adjacent region(s) by comparing data in the set of aligned data (some or all of the data may be compared) using a machine learning model; and producing an output containing information relating to the subject's heart.

In a third aspect, there is a system for identifying changes in a subject's heart or an adjacent region over time, the system comprising: a memory comprising instruction data representing a set of instructions; and one or more processors configured to communicate with the memory and to execute the set of instructions, wherein the set of instructions, when executed by the processor cause the processor to carry out the aforementioned computer implemented method.

It should be understood that when referring to the processing that takes place in the method it is not limited the method to being carried out by a single processor or computer. Instead, the method may be carried out by plural processors which may be provided at different locations and interconnected, for example, using a distributed network. An example of this may be the processing of images from multiple data sources that have been transferred or stored on a distributed network (e.g. a "cloud"-based network). Furthermore, any processing stages may occur on different processors within a network.

A processor may be implemented in any suitable manner, with software and/or hardware, to perform the various functions required. Examples of processor components that may be employed in various embodiments of the present disclosure include, but are not limited to, conventional microprocessors, application specific integrated circuits (ASICs), and field-programmable gate arrays (FPGAs). The processor may be associated with one or more non-transitory storage media such as volatile and non-volatile computer memory such as RAM, PROM, EPROM, and EEPROM. The non-transitory storage media may be encoded with one or more programs that, when executed on one or more processors and/or controllers, perform the required functions. Various storage media may be fixed within a processor or controller or may be transportable, such that the one or more programs stored thereon can be loaded into the signal processing unit, property determination unit and/or processor.

In embodiments, the system includes a user interface, such as a display, for displaying the output. Alternatively or additionally, the system may include a communications interface device, such as a wireless transmitter, configured to transmit the output to an external device, such as a personal computer, tablet, smartphone, remote server, etc.

In an embodiment, the system includes imaging device adapted to collect a set of imaging data relating to a subject's heart, for instance an ultrasound device, a CT device or a MRI device.

These and other aspects of the invention will be apparent from and elucidated with reference to the embodiment(s) described hereinafter.

It should be understood that the claims, when referring to the processing that takes place in the method, do not limit the method to being carried out by a single processor or computer. Instead, the method may be carried out using a distributed network. An example of this may be the processing of images from multiple data sources that have been transferred or stored on a distributed network (e.g a "cloud"-based network). Furthermore, any processing stages may occur on different processors within a network.

It will be appreciated by those skilled in the art that two or more of the above-mentioned options, implementations, and/or aspects of the invention may be combined in any way deemed useful.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows schematic diagram of a computer implemented method according to the invention; and
Figure 2 shows schematic diagram of a computer implemented method according to the invention; and
Figure 3 shows schematic diagram of a method according to the invention.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfil the functions of several items recited in the claims. The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

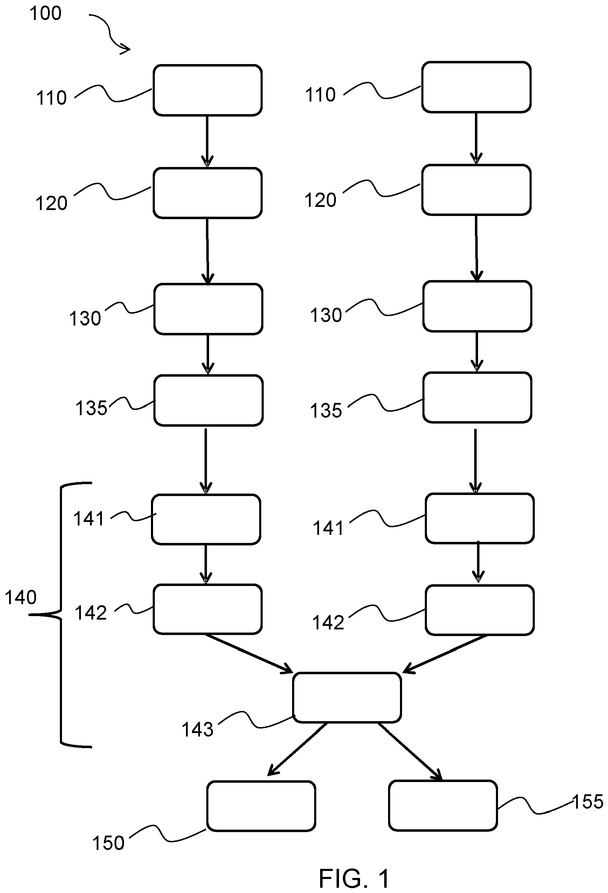

Figure 1 illustrates a computer implemented method of predicting early onset heart failure. The method 100, comprises of a series of steps that are represented by blocks. In Figure 1 there are two parallel blocks for the pre-analysis steps. These two parallel blocks represent different studies (i.e. image gathering events, such as scans) that were taken at different points in time, each of which comprises an image of the patient's heart. Although there are only two parallel blocks in Figure 1, this method can easily be extended to any number of image scans or frames that form part of a scan that have been taken at a plurality of points in time.

Each branch of the method 100 comprises receiving imaging data comprising an image or multiple frames of a subject's heart 110 from an image as an input. The images are taken from the same imaging modality, for example ultrasound, CT or MRI.

Each image is then converted 120 to a heart model, which in this embodiment is through the use of model-based segmentation 120. Model based segmentation 120 is able to segment a heart shown in an image and generate a corresponding anatomical model or mesh of the heart. In the case that a mesh is produced, the mesh may be anatomically plausible. This process is applied to every image, which can include applying this to every frame within an imaging scan or every image scan which results in the provision of a time series of heart models. However, as the input data (i.e. the images) have been obtained over plural points in time, there may be large variations between the images. For example, caused by the use of different devices, different acquisition parameters, and operator dependencies.

Alignment 130 of the anatomical models can then occur. In this embodiment, the heart models are converted into aligned data so as to form a set of aligned anatomical models.

Alignment in this embodiment comprises mapping the heart models onto a common anatomical coordinate system based on anatomical landmarks that are present in all of the heart models or meshes. A time series of anatomical representations that have been mapped onto a single coordinate system can therefore be obtained. The process of alignment 130 is what enables subtle changes in different regions of the heart to be identified.

The aligned data in the form of aligned heart models are then converted into a graph 135. This is an encoding step in which the data from the aligned heart models is extracted and a corresponding graph is produced. The graph encodes key features and information from the heart model, such as at least one set of coordinates in an anatomical coordinate frame, geometric features, anatomical region codes, and neighboring image intensities, or combinations thereof. The conversion of aligned data in the form of aligned heart models results in a series of graphs, in this embodiment with each graph corresponding to each image. The key features are encoded into the graph's nodes and edges.

The method 100 subsequently comprises processing the resultant graphs so as to identify changes in at least one region of the subject's heart 140 by comparing the graphs using a machine learning model and producing an output containing information relating to the subject's heart 150.

In this embodiment, the step of processing the graphs using a machine learning model comprises several sub-steps. The first step of the machine learning model-based identification 140 comprises processing using a convolutional neural network (CNN) 141 (such as a graph- or mesh-based CNN, e.g. MeshCNN) to extract anatomical features from the graphs. The result of the mesh CNN is a feature vector capturing anatomical information for each image.

The step of identification 140 further comprises two recurrent processing stages 142, 143. The recurrent processing steps 142, 143 can be carried out using e.g. gated recurrent units (GRUs), long short-term memory (LSTM) cells, or other recurrent models.

In this embodiment, the processing considers motion trajectories. The first recurrent processing step 142 comprises short-term temporal processing of the feature vectors to capture local motion anomalies e.g. within a cardiac cycle. By this it is meant that this first processing step 142 considers motion determined by image(s) taken within a single study obtained at a single point in time. One particular implementation of the machine learning model in this embodiment uses a short term LSTM.

In this embodiment, the second recurrent processing step 143 is then used to compare the per-study features so as to compare all of the studies taken at different points in time. This captures long-term changes of motion trajectories that could indicate future heart failure. This processing stage 143 uses a long-term LSTM.

The method then comprises providing an output 150, which in this embodiment is a prediction of whether the subject might suffer from heart failure in the future. This is based on the output of the long term LSTM processing 143 and may comprise a model.

In this embodiment, the method may also comprise producing a saliency map 155. The most important studies, sequences, and frames or images are presented to the user, based on the level of relevance (saliency). The saliency map may provide representations of the heart (e.g. frames or images), with the most important anatomical region highlighted.

Saliency maps with guided backpropagation can be used which highlight the most important regions in the input data. The saliency map can visualize and highlight the important time points and anatomical regions. This may be achieved by framing regions in a study. In one embodiment, the user would see a sequence of heart graphs with a heat map at certain time points on top of certain anatomies. Thus, a clinician that uses the model can be guided to important time points and anatomical regions that are relevant for predicting early onset heart failure. The conversion of images to aligned graphs enable the saliency map to point at specific anatomical regions of interest.

In the embodiments, the runtime to produce the model may be reduced by using a mechanism such as frame propagation with short term ultrasound sequences. Frame propagation involves propagating a produced mesh from one frame to the next, within one ultrasound study.

In the above embodiments, motion trajectory analysis is carried out; however, in other embodiments this may not be carried out. Identification or analysis of the changes that occur in the heart over time may track other features such as changes in wall thickness and image intensity may be used to track how changes occur over time.

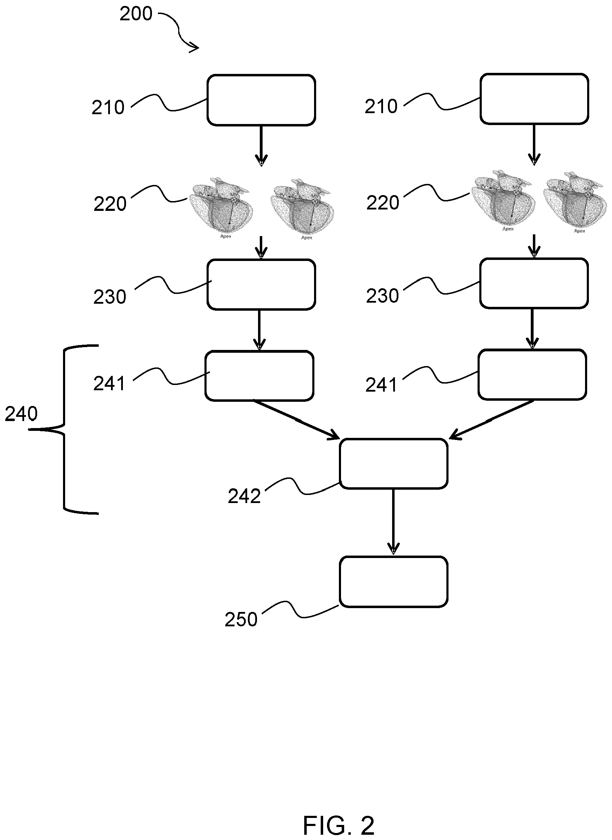

Figure 2 illustrates a further embodiment of the computer implemented method of predicting early onset heart failure. The method 200 comprises a series of steps that are represented by blocks.

The method comprises first receiving imaging data 210. After this, the imaging data is converted into a mesh 220 by means of model based segmentation. A mesh can be understood to be a type of model. This mesh is then converted into a graph 230.

In contrast to the step of conversion in Figure 1, the conversion of the model of the heart into a graph 230 that is aligned in the present method does not comprise a distinct stage of producing a set of aligned model prior to extracting the data from the models. Instead, alignment occurs during the extraction and generation of the graphs so as to produces a set of aligned graphs. The alignment 230 may therefore occur alongside the identification and production of one of: geometric features, anatomical region codes, and neighboring image intensities for the graphs.

The processing also differs from that of Figure 1. In this case, the identification of differences 240 is carried out by neural networks. Specifically, the. undergo processing via a convolutional neural network 241 (CNN) in order to extract anatomical features from the graphs. The result of the CNN is a feature vector capturing anatomical information for each graph. A gated recurrent unit (GRU) then processes the sequential data 242 and an output containing information relating to a subject's heart 250 is produced.

The output can be in a number of forms. It may comprise an indication of health (such as diagnosis) or may comprise a prediction regarding the onset of a disease (e.g. expected progression). It may comprise a medical image or model indicating regions of particular interest or similar. The output may be provided on a display of an associated system.

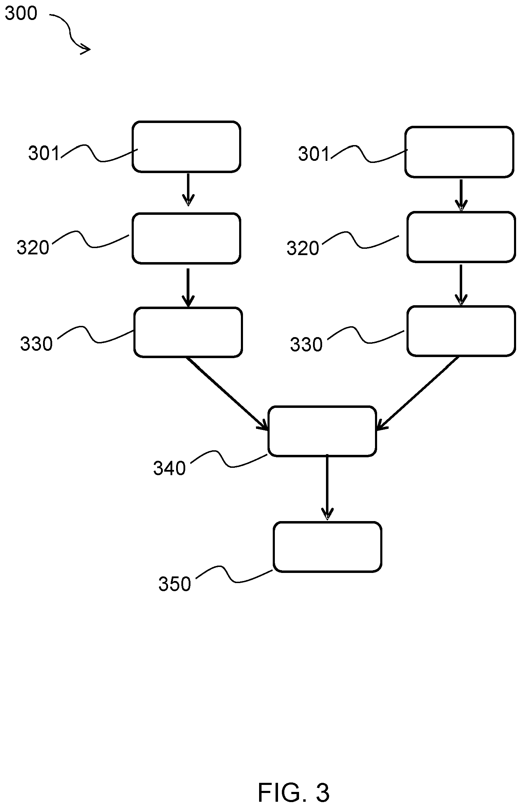

Figure 3 illustrates a method 300 of identifying changes in a subject's heart overtime.

In this embodiment the method 300 comprises the step of obtaining image at a plurality of points in time relating to a subject's heart 301. This comprises carrying out plural studies or investigations at a number of different points in time to generate the required images. This may include, for example, ultrasound studies, CT studies or MRI studies. The medical imaging data compared in the above-mentioned methods and the present method are obtained from the same modality. The medical imaging data may be in the form of 2D, 3D, 2D + t, or 3D + t data (such as ultrasound, CT or MRI imaging data).

The subsequently analysis is then in accordance with the computer-implemented methods described herein.

For example, an anatomical model is then generated of a subject's heart 320. The anatomical models are aligned to form aligned data 330. A graph is then generated by extracting the data from the aligned data (models). This graph is then processed by a machine learning model 340 and an output is then produced with a prediction regarding the onset of a heart condition 360.

Although the invention has been disclosed in the context of specific embodiments, it will be appreciated that these may be modified without departing from the invention.

For example, although this invention is applicable to ultrasound (e.g. cardiac ultrasound), other imaging modalities may be used.

The computer implemented methods may be carried out on a single processor, or may be across plural processed (for example a cloud-based platform or distributed network).

Similarly, although a two-step processing model, including e.g. LSTM, has been used in the embodiment depicted in Figure 1 the processing of each graph, it will be appreciated that other machine learning algorithms or models could be used. For example, other recurrent neural network models that include one or several layers may be used at this stage. In an embodiment, a different model containing one or multiple temporal deep stages may be used.

In the embodiments, the model based segmentation that produces a mesh may be enhanced by or replaced by a deep-learning model. All other processing steps would remain the same.

In a further embodiment, short-term and long-term temporal processing may be performed with other temporal processing methods such as convolutional models or transformer-based models.

In the above depicted embodiments, each of the images in the imaging data were used to generate models, each of the models were aligned and data from each of the aligned models were used at the basis of the comparison; however, it will be appreciated that in other embodiments only a part of the data may be used, such as a plurality of images (but not necessarily all (each) of the images). The same applies to the subsequent steps.

**Claims**

1. A computer implemented method of identifying changes in a subject's heart or an adjacent region over time, the method comprising:

    receiving a set of imaging data relating to a subject's heart, the set of imaging data comprised of images of the subject's heart obtained at a plurality of points in time;
    generating an anatomical model of the subject's heart for a plurality of the images in the set of imaging data so as to provide a set of anatomical models of the subject's heart corresponding to a plurality of points in time; and
    aligning a plurality of the anatomical models in the set of anatomical models relative to one another so as to provide a set of aligned data, wherein the set of aligned data can be used to identify changes in at least one region of the subject's heart by comparing data in the set of aligned data using a machine learning model.

2. The method according to claim 1, further comprising:

    identifying changes in at least one region of the subject's heart by comparing data in the set of aligned data using the machine learning model; and
    generating an output containing information relating to the subject's heart.

3. The method according to claim 2, wherein the output is a prediction relating to an onset of a cardiovascular condition.

4. The method according to claim 2 or claim 3, wherein the steps of aligning a plurality of the anatomical models in the set of anatomical models and identifying changes in at least one region of the subject's heart by comparing the data in the set of aligned data using a machine learning model comprises:

    extracting data relating to the at least one region of the subject's heart from each of a plurality of the anatomical models;
    generating a graph representative of the extracted data for each of the plurality of anatomical models; and
    comparing the graphs for each of the plurality of anatomical models.

5. The method according to claim 4, wherein the step of aligning a plurality of the anatomical models in the set of anatomical models relative to one another is carried out prior to extracting the data relating to the at least one region of the subject's heart such that the extracted data is extracted aligned data.

6. The method according to claim 4, wherein the step of aligning a plurality of the anatomical models in the set of anatomical models relative to one another is carried out after extracting the data relating to the at least one region of the subject's heart such that the extracted data is subsequently aligned.

7. The method according to any of claims 4 to 6, wherein the step of extracting data relating to the at least one region of the subject's heart from each of the plurality of the anatomical models comprises at least one of: a coordinate frame associated with the region of the subject's heart, geometric features, anatomical region codes and image intensities.

8. The method according to any of claims 4 to 7, wherein the machine learning model identifies changes in the at least one region of the subject's heart by comparing the graphs for each of the plurality of the anatomical models using a recurrent processing unit.

9. The method according to claim 8, wherein the recurrent processing unit performs temporal processing within a cardiac cycle; and/or identifies changes of motion trajectories.

10. The method according to any preceding claim, wherein aligning a plurality of the anatomical models in the set of anatomical models relative to one another so as to provide the set of aligned data comprises:

    defining a coordinate frame for the plurality of the anatomical models based on at least one identifiable anatomical feature common in each of the plurality of the anatomical models; and
    aligning the representations by aligning the coordinate frame of each of the plurality of the anatomical models.

11. The method according to any preceding claim, wherein the output comprises an indication of the input data or a part of the input data on which the output has been based.

12. The method according to any proceeding claim, wherein the images comprise ultrasound images.

13. A method of identifying changes in a subject's heart or an adjacent region over time, the method comprising:

obtaining images of a subject's heart at a plurality of points in time to produce a set of imaging data;
generating an anatomical model of the subject's heart for a plurality of the images in the set of imaging data so as to provide a set of anatomical models of the subject's heart corresponding to a plurality of points in time; and
aligning a plurality of the anatomical models in the set of anatomical models relative to one another so as to provide a set of aligned data, wherein the set of aligned data is for identifying changes in at least one region of the subject's heart by comparing data in the set of aligned data using a machine learning model.

14. The method of claim 13, further comprising:

identifying changes in the at least one region of the subject's heart by comparing data in the set of aligned data using a machine learning model; and
producing an output containing information relating to the subject's heart.

15. A system for identifying changes in a subject's heart or an adjacent region overtime, the system comprising:

a memory comprising instruction data representing a set of instructions;
one or more processors configured to communicate with the memory and to execute the set of instructions, wherein the set of instructions, when executed by the one or more processors cause the one or more processors to carry out the computer implemented method of any of claims 1 to 12.

FIG. 1

200

210

210

220 220

230 230

241 241

240

242

250

FIG. 2

300

301

320

330

301

320

330

340

350

FIG. 3

Europäisches
Patentamt

European
Patent Office

Office européen
des brevets

# EUROPEAN SEARCH REPORT

Application Number

EP 21 21 5215

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/350179 A1 (BELLO GHALIB A [GB] ET AL) 11 November 2021 (2021-11-11)<br>* paragraph [0001] *<br>* paragraph [0045] *<br>* paragraph [0080] – paragraph [0081] *<br>* paragraph [0043] *<br>* paragraph [0178] *<br>* paragraph [0158] *<br>* paragraph [0146] – paragraph [0151] *<br>* paragraph [0181] *<br>* paragraph [0094] *<br>* paragraph [0048] *<br>----- | 1-15 | INV.<br>G16H30/40 |
| X | GHALIB A BELLO ET AL: "Deep learning cardiac motion analysis for human survival prediction",<br>ARXIV.ORG, CORNELL UNIVERSITY LIBRARY, 201 OLIN LIBRARY CORNELL UNIVERSITY ITHACA, NY 14853,<br>8 October 2018 (2018-10-08), XP080930814,<br>* the whole document *<br>----- | 1-15 | |
| X | US 2010/280352 A1 (IONASEC RAZVAN IOAN [US] ET AL) 4 November 2010 (2010-11-04)<br>* paragraph [0018] *<br>* paragraph [0020] – paragraph [0028] *<br>* paragraph [0032] *<br>* paragraph [0046] *<br>* paragraph [0047] *<br>----- | 1-15 | TECHNICAL FIELDS SEARCHED (IPC)<br><br>G16H |
| X | EP 3 444 777 A1 (SIEMENS HEALTHCARE GMBH [DE]) 20 February 2019 (2019-02-20)<br>* paragraph [0006] – paragraph [0008] *<br>* paragraph [0037] – paragraph [0043] *<br>----- | 1-15 | |
| X | US 2010/070249 A1 (IONASEC RAZVAN [US] ET AL) 18 March 2010 (2010-03-18)<br>* paragraph [0026] *<br>* paragraph [0027] – paragraph [0032] *<br>----- | 1-15 | |

The present search report has been drawn up for all claims

| Place of search | Date of completion of the search | Examiner |
|---|---|---|
| Munich | 30 May 2022 | Megalou-Nash, M |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 21 5215

This annex lists the patent family members relating to the patent documents cited in the above-mentioned European search report.
The members are as contained in the European Patent Office EDP file on
The European Patent Office is in no way liable for these particulars which are merely given for the purpose of information.

30-05-2022

| Patent document cited in search report | | Publication date | Patent family member(s) | | Publication date |
|---|---|---|---|---|---|
| US 2021350179 | A1 | 11-11-2021 | EP | 3861560 A1 | 11-08-2021 |
| | | | US | 2021350179 A1 | 11-11-2021 |
| | | | WO | 2020070519 A1 | 09-04-2020 |
| US 2010280352 | A1 | 04-11-2010 | NONE | | |
| EP 3444777 | A1 | 20-02-2019 | CN | 109427058 A | 05-03-2019 |
| | | | EP | 3444777 A1 | 20-02-2019 |
| | | | US | 2019057505 A1 | 21-02-2019 |
| US 2010070249 | A1 | 18-03-2010 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VASWANI, A. ; SHAZEER, N. ; PARMAR, N. ; USZ-KOREIT, J. ; JONES, L. ; GOMEZ, A. N. ; POLO-SUKHIN, I.** Attention is all you need. *Advances in neural information processing systems,* 2017, 5998-6008